(19) **Europäisches Patentamt / European Patent Office / Office européen des brevets**

(11) **EP 3 415 170 B1**

(12) **EUROPEAN PATENT SPECIFICATION**

(45) Date of publication and mention
of the grant of the patent:
**22.04.2020 Bulletin 2020/17**

(21) Application number: **17750374.5**

(22) Date of filing: **10.02.2017**

(51) Int Cl.:
*A61L 15/28* (2006.01)          *A61F 13/00* (2006.01)
*C08B 11/12* (2006.01)

(86) International application number:
**PCT/JP2017/004995**

(87) International publication number:
**WO 2017/138653 (17.08.2017 Gazette 2017/33)**

(54) **SHEET FOR COVERING WOUND**

FOLIE ZUR WUNDABDECKUNG

FEUILLE PERMETTANT DE RECOUVRIR UNE PLAIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.02.2016 JP 2016023491**

(43) Date of publication of application:
**19.12.2018 Bulletin 2018/51**

(73) Proprietor: **Asahi Kasei Kabushiki Kaisha
Tokyo 100-0006 (JP)**

(72) Inventor: **HARA, Yuichi
Tokyo 101-8101 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(56) References cited:
**WO-A1-2011/121858      WO-A1-2013/129584
JP-A- 2008 285 611      JP-A- 2013 135 704
JP-A- 2015 070 944      US-A1- 2008 147 026**

**Description**

FIELD

The present invention relates to a sheet for covering a wound comprising natural or regenerated cellulose fibers including at least partially protonated carboxymethyl cellulose (hereinafter abbreviated as CMC). More specifically, the present invention relates to a sheet for covering a wound comprising CMC that can maintain high morphological stability while maintaining high liquid absorbency, even after liquid absorption.

BACKGROUND

When a wound occurs on the skin or the like of a human, a wound protection material such as a surgical dressing or a wound covering material is used to protect the site of the wound.
Patent Document 1 below describes a soluble vulnerary hemostatic cellulose fiber in which the degree of substitution of the CMC is from 0.5 to less than 1.0 and describes that the CMC has a cell adhesion promoting action. Furthermore, Patent Document 2 below describes that when CMC is applied to the site of a wound, insoluble foreign matter which causes a risk of inflammation or the like of the wound site does not remain.
Sodium carboxymethyl cellulose (hereinafter abbreviated as CMC-Na) in which sodium is bonded with the carboxymethyl group is commonly used as CMC for medical use. However, though CMC-Na has very high liquid absorbency, it has a problem of low strength. In wound healing, it is necessary that, after the exudate coming out from the wound is retained and the wound has been treated, the wound covering sheet be removable in a state in which the shape is maintained. Furthermore, since the wound site may become inflamed if the substrate remains, the substrate should not remain in the wound site.
A protonated carboxymethyl cellulose (hereinafter abbreviated as CMC-H) in which a proton is bonded to a carboxymethyl group by immersing the CMC-Na in acetic acid, nitric acid, hydrochloric acid or the like having a predetermined concentration, has also been developed. While CMC-H has increased strength, it has a problem that the liquid absorbency thereof is similar to that of ordinary cellulose nonwoven fabric. The following Patent Document 3 uses partially protonated CMC as an adhesion prevention material but has poor absorbency and cannot be used for wound healing.
JP 2013-135704 discloses a hemostatic material containing carboxymethylcellulose having a degree of substitution of hydroxyl groups between 0.5 and 1.0. It also discloses that 70 % or more of the carboxymethyl groups are of the type. Said hemostatic material is in a non-woven fabric and then sheets have a thickness of 0.26 mm. The non-woven fabric has a basis weight of 10 to 40 g/m$^2$.

[PRIOR ART DOCUMENTS]

[PATENT DOCUMENTS]

[Patent Document 1] Japanese Unexamined Patent Publication (Kokai) No. 2000-256958
[Patent Document 2] Japanese Unexamined Patent Publication (Kokai) No. 2002-143210
[Patent Document 3] WO 2011/121858

SUMMARY

PROBLEM TO BE SOLVED BY THE INVENTION

In view of the state of the art described above, the problem to be solved by the present invention is to provide a novel sheet for covering a wound having high liquid absorbency and strength.

[MEANS FOR SOLVING THE PROBLEM]

The present inventors have discovered that a carboxymethylated CMC in which the degree of protonation is controlled can be prepared by immersing a structure containing natural or regenerated cellulose fibers in an acid, such as acetic acid or hydrochloric acid, which has been adjusted to a predetermined concentration, and have further discovered that by controlling the degree of substitution and the degree of protonation of the CMC to within a predetermined range, strength can be improved in a state in which the liquid absorbency is maintained. Based on these findings, the present invention has been achieved.

In other words, the present invention is as described below.

[1] A sheet for covering a wound, which is a wound covering material containing fibers of carboxymethylated natural or regenerated cellulose, wherein the average degree of substitution of hydroxyl groups in a glucose unit constituting the cellulose is in the range of 0.3 to 1.0, and 5% to 60% of carboxymethyl groups are protonated.
[2] The sheet for covering a wound according to [1], having a thickness in the range of 0.03 mm to 5.0 mm.
[3] The sheet for covering a wound according to [1] or [2], having a density of 0.03 g/cm$^3$ to 1.0 g/cm3.
[4] The sheet for covering a wound according to any one of [1] to [3], in a cotton-like, woven fabric, or non-woven fabric form.

EFFECTS OF THE INVENTION

By performing wound healing using the sheet for covering a wound according to the present invention, wound healing is promoted by retaining more exudate by the CMC, further, the morphology is retained during treatment, and furthermore, few residuals remain in the wound, whereby inflammation of the wound is prevented.

EMBODIMENTS FOR CARRYING OUT THE INVENTION

The embodiments of the present invention will be described in detail below.
In the present specification, "sheet for covering a wound" indicates a sheet-like structure that protects wounds, prevents external infection, accelerates healing of the wound, and relieves pain. For example, the sheet for covering a wound may be adhesive bandages or wound covering materials used for moist wound healing for pressure sores, burns, or the like, but the structure is not limited thereto.
The natural or regenerated cellulose fibers of the present embodiment are not specifically limited, and known cellulose fibers such as cuprammonium rayon, viscose rayon, cotton, pulp, and polynosic, preferably cuprammonium rayon or viscose rayon, and more preferably cuprammonium rayon may be used. These fibers may be either continuous long fibers (filaments) or short fibers (staple fibers). Long fibers are preferably continuous long fibers. In the present specification, "long fibers" means fibers having a length of 10 mm or more, preferably having a fiber length of 20 mm or more, more preferably 50 mm or more, and still more preferably are continuous length fibers.
As the form of the structure of the present embodiment, a cotton-like, woven fabric, or nonwoven fabric form is preferable. A woven or nonwoven fabric of regenerated cellulose fibers is a more preferable form, a nonwoven fabric of regenerated cellulose fibers is a further preferable form, and a cupra nonwoven fabric is an even further preferable form. For a nonwoven fabric form, since the fibers are entangled at the time of wetting even in a gelled state, it is easy for the nonwoven fabric to achieve moderate flexibility while maintaining the strength at the time of wetting, and the fabric easily follows the movement of the skin when attached thereto. Furthermore, it is easy to adhere the wound covering material along indented portions. Since the surface area thereof is large, it is possible to quickly absorb exudate coming out of the wound. If the fibers constituting such a nonwoven fabric are cupra, since the degree of crystallinity thereof is low, the reactivity during carboxymethylation is high and the morphological stability is Excellent. In the case of a nonwoven fabric, binder-free nonwoven fabrics are preferred since the permeation rate of the solution in a nonwoven fabric to which a binder has been imparted is slow and dissolution of the binder component is a concern.
Furthermore, in addition to natural or regenerated cellulose fibers, fibers other than natural or regenerated cellulose fibers, for example, synthetic fibers such as polyester fibers, polypropylene fibers, nylon fibers, etc., may be included in the sheet-like structure of the present embodiment. Such synthetic fibers may be either long fibers or short fibers.
The density (g/cm3) of the sheet-like structure of the present embodiment is calculated by (the weight of the nonwoven fabric (g) / volume of the nonwoven fabric (cm3) and is preferably in the range from 0.03 g/cm$^3$ to 1.0 g/cm$^3$, more preferably in the range from 0.03 g/cm$^3$ to 0.5 g/cm$^3$, and most preferably 0.03 g/cm$^3$ to 0.4 g/cm$^3$. If the density is too low, it is difficult to achieve sufficient strength even if partially protonated. Conversely, if the density is too high, deformation becomes difficult, whereby placement on an effected part becomes complex.
For woven or nonwoven fabrics, the thickness of the sheet-like structure of the present embodiment is preferably in the range from 0.03 mm to 5.0 mm, more preferably in the range from 0.03 mm to 3.0 mm, further preferably in the range from 0.03 mm to 1.0 mm, and most preferably in the range from 0.03 mm to 0.8 mm. If the thickness is too thin, it is difficult to achieve sufficient strength even if partially protonated. Conversely, if the thickness is too thick, flexibility is eliminated, whereby placement on an effected part becomes complex. In the present specification, for example, the "thickness" of a nonwoven fabric means the value obtained by measuring with a load of 1.96 kPa by the thickness test according to JIS-L 1096.
It is necessary that the sheet-like structure of the present embodiment have an average degree of substitution (DS) of hydroxyl groups in a glucose unit constituting the cellulose when carboxymethylated in the range from 0.3 to 1.0, preferably from 0.3 to less than 0.8, and more preferably from 0.3 to less than 0.6. If DS is 0.3 or more, when partially protonated, sufficient liquid absorbency can be ensured. However, when the degree of substitution exceeds 1.0, sufficient strength cannot be obtained, even if protonation is performed.

Furthermore, although the terminals of the carboxymethyl groups become sodium salt at the time of carboxymethylation, a part of the carboxymethyl groups can be protonated by treating with an acid to obtain the sheet-like structure of the present embodiment. In order to obtain sufficient strength while maintaining high liquid absorbency, it is necessary that the degree of protonation of the carboxymethyl groups at that time be in the range of 5% to 60%, and preferably 10% to 60%.

The continuous long fiber cupra non-woven fabric can be produced by the following method.

A stock solution prepared by dissolving a cotton linter, from which foreign matter has been removed and which has a controlled degree of polymerization in a copper ammonium solution, is extruded from a spinneret having pores (raw stock solution discharge holes), dropped into a funnel along with water, solidified by deammoniation, stretched and shaken off onto a net to form a web. Thereafter, by vibrating the net in the direction perpendicular to the direction of travel while advancing the net, the fibers shaken off of the net draw a sine curve. 100 to 500 times stretching at the time of spinning can be achieved and it is possible to arbitrarily adjust the stretching ratio by changing the shape of the spinning funnel and the amount of spinning water flowing down into the spinning funnel. By changing the stretching ratio, it is possible to change the single-fiber fineness and strength of the nonwoven fabric. Furthermore, it is also possible to control the trace amounts of low molecular weight cellulose, so-called "hemi-cellulose", remaining in the stock solution by changing the amount of spinning water or the temperature. By controlling the traveling speed and the vibration amplitude of the net, it is possible to control the fiber orientation direction, whereby the strength, elongation, etc., of the nonwoven fabric can be controlled.

The carboxymethylated sheet-like structure can be produced by the following method.

First, a structure of natural or regenerated cellulose fibers is stirred in an alcohol-containing sodium hydroxide aqueous solution at 35 °C for 30 minutes while maintaining an alkaline state. After discharging the reagent in the reaction vessel, an alcohol-containing sodium monochloroacetate is added and stirred at 30 °C to 50 °C for 1 to 12 hours. At that time, the degree of substitution is controlled by controlling the bath ratio of the reaction solution and the structure, the temperature, and the time. Furthermore, other reaction conditions can be appropriately changed in consideration of production costs and the like. The obtained structure is adjusted to pH 6.0 to 8.0 with an acetic acid-containing ethanol aqueous solution, and alcohol replacement is then carried out with 70 wt%, 90 wt%, and 100 wt% ethanol. Since the structure will harden if it contains even trace amounts of water, by gradually increasing the alcohol concentration, it is possible to reliably perform alcohol substitution, whereby morphological stability can be maintained. Subsequently, the structure is immersed in an acid-containing ethanol solution adjusted to a predetermined concentration, stirred for 1 hour, alcohol substituted with 70 wt%, 90 wt%, and 100 wt% ethanol and dried to obtain a protonated sheet-like structure. The step of immersion in acid may be performed simultaneously with the neutralization step. In other words, generally two steps are required to carry out the acid immersion step after the neutralization step, but the neutralization step and the acid immersion step may be performed at the same time and may be performed in one step.

The method for partially protonating the sheet-shaped structure of the present embodiment is not particularly limited, and the sheet-shaped structure is preferably protonated by submerging in acetic acid, hydrochloric acid, or nitric acid adjusted to a predetermined concentration using alcohol, more preferably acetic acid adjusted to a predetermined concentration. For protonation using acetic acid, a reactor made of SUS can be used.

The sheet-like structure of the present embodiment may be used directly after the partial protonation followed by drying, but is preferably heat treated at a temperature of 50 °C or more for 1 h or more, more preferable at a temperature of 80 to 120 °C for 3 h or more, and more preferably at a temperature of 100 to 120 °C for 3 h or more. By performing heat treatment, the orientation of the molecules is optimized and the strength can be further increased by increasing the intramolecular hydrogen bonding thereof. As the heat treatment method, hot air treatment, dry heat treatment, wet heat treatment, vacuum heat treatment, or the like may be used. Hot air treatment is preferable for efficient processing, but the method of heat treatment is not limited thereto.

The sheet-like structure of the present embodiment is gelled even in a state where high strength is maintained at the time of wetting. In addition to having high liquid absorbing properties by gelling upon wetting, skin adhesion increases, and followability with the movement of the skin is good. Furthermore, gelation softens the texture thereof and reduces the impact on the skin.

The methods for measuring the physical property values of the CMC fiber sheet-like structure will be described below.

1. Measurement of the Average Degree of Substitution

(i) Acidity and Alkalinity

About 1 g of sample (anhydrous) is precisely weighed in a 300 ml Erlenmeyer flask, to which 200 mL of water is added to dissolve the sample. 5 mL of 0.05 mol/L sulfuric acid is added thereto with a pipette, this mixture is boiled for 10 minutes, cooled, a phenolphthalein indicator is added thereto, and the mixture is titrated with 0.1 mol/L potassium hydroxide (S mL). Simultaneously, a blank test is performed (B mL), and the following formula (1) is calculated:

$$\text{Alkalinity} = \{(B - S) \times f\} / \text{sample anhydrous weight (g)} \ \dots \text{Formula (1)},$$

where f = 0.1 mol/L potassium hydroxide titer.
When the value of the expression $(B - S) \times f$ is negative, the alkalinity should be replaced with acidity.

(ii) Average Degree of Substitution

0.5 to 0.7 g of sample (anhydrous) is precisely weighed, wrapped in filter paper and incinerated in a porcelain crucible. After cooling, the sample is transferred to a 500 mL beaker, about 250 mL of water is added thereto, 35 mL of 0.05 mol/L sulfuric acid is added thereto with a pipette, and the mixture is boiled for 30 minutes. After cooling, a phenolphthalein indicator is added and counter-titration of the excess acid with 0.1 mol/L potassium hydroxide is performed. The degree of substitution is calculated by the following formulas (2) and (3):

$$A = (a \times f - b \times f1) / \text{sample anhydrous weight (g)} - \textbf{alkalinity} \ (\text{or} + \text{acidity}) \ \dots \text{Formula (2)},$$

$$\text{Degree of Substitution} = (162 \times A) / (10000 - 80 \times A) \ \dots \text{Formula (3)},$$

where, A = the amount (mL) of 0.05 mol/L sulfuric acid consumed in the bound alkali in 1 g of the sample, a = use amount (mL) of 0.05 mol/L sulfuric acid, f = 0.05 mol / L sulfuric acid titer, b = titration amount (mL) of 0.1 mol/L potassium hydroxide, and fl = 0.1 mol/L potassium hydroxide titer. The average value thereof (N = 3 or more) is set as the average degree of substitution.

2. Degree of Protonation

The sheet-like structure is cut into an area of 1 cm$^2$ or more. Thereafter, the cut portion is placed into an FT-IR (ATR) apparatus and surface analysis is performed. Subsequently, ATR correction, baseline correction and normalization are performed, the peak height at a wavelength of 1590 cm$^{-1}$ is measured, and the degree of protonation is calculated from the ratio of the peak heights before and after protonation by the following formula (4):

$$\text{Degree of Protonation (\%)} = (A - B) / A \times 100 \ \dots \text{Formula (4)},$$

where A = (1590 cm$^{-1}$ peak height of the sample before protonation) - (1590 cm$^{-1}$ peak height of the sample after treatment with 5 wt% hydrochloric acid for 1 hour), and B = (1590 cm$^{-1}$ peak height of the sample after protonation) - (1590 cm$^{-1}$ peak height of the sample after treatment with 5 wt% hydrochloric acid for 1 hour). An equivalent sample is prepared as the sample before protonation.

3. Liquid Absorbency (Amount (g/100 cm$^2$))

The liquid absorbency of the sheet-like structure is measured for absorption capacity when freely swelled according to EN 13726-1. Specifically, the sheet-like structure is cut into a 5 cm × 5 cm square and placed in a petri dish. After heating simulated exudate (described in EN 13726-1) in an amount equal to 40 times the weight of the sample to 37 °C, the simulated exudate is then added thereto and left in an incubator at 37 °C for 30 minutes. Thereafter, the amount of liquid absorption is calculated from the weight before and after incubation by the following formula (5):

$$\text{Liquid Absorption Amount (g/100 cm}^2) = (A - B) \times 4,$$

where A = weight (g) in a dry state prior to immersion, and B = weight (g) after incubation for 30 minutes.

4. Strength

The sheet-like structure is cut into a 5 cm × 5 cm square and placed in a petri dish. Thereafter, the simulated exudate in an amount equal to 40 times the weight of the sample is added thereto, and allowed to sit for 30 minutes at 37 °C. The sample is then removed, held with tweezers, and the strength is evaluated with the following criteria:

Poor: shape is not maintained
Fair: shape is maintained but it is difficult to hold
Good: shape is maintained, it can be held, but disintegrates when pulled strongly
EXC: can withstand a tensile force of a certain degree of strength

5. Shape Retention (at the Time of Long-Term Immersion)

The sheet-like structure is cut into a 5 cm × 5 cm square and placed in a petri dish. Thereafter, the simulated exudate in an amount equal to 40 times the weight of the sample is added thereto and allowed to sit for 3 days at 37 °C. Subsequently, the shape of the sample is observed and the strength is evaluated according to the following evaluation criteria:

Poor: shape is not maintained
Fair: shape is maintained but disassociated fibers disintegrate and disperse with light shaking
Good: shape is maintained

EXAMPLES

Examples of the present invention will be specifically described below.

[Reference Example 1]

100 g of a regenerated cellulose continuous long-fibers sheet-like structure (cupra sheet-like structure) (width: 20 cm, basis weight: 80 g/m², thickness: 0.5 mm, density: 0.154 g/m³), Lyocell short-fibers nonwoven fabric, or rayon short-fiber nonwoven fabric was placed in a reaction vessel. Thereafter, a sodium hydroxide-containing ethanol aqueous solution (water: 875 g, ethanol: 875 g, NaOH: 162.5 g) was added thereto and stirred for 30 minutes at 35 °C. Next, after discharging the reagent in the reaction vessel, an aqueous ethanol solution containing sodium monochloroacetate (water: 300 g, ethanol: 960 g, sodium monochloroacetate: 122.5 g) was added and stirred at 30 or 50 °C for 1 to 12 hours and was then dried to obtain a carboxymethylated sheet-like structure. After adjusting the pH of the obtained sheet-like structure described above to pH 6.0 to 8.0 with an acetic acid-containing ethanol solution (acetic acid: 37.5 g, distilled water: 375 g, ethanol: 875 g), the sheet-like structure was washed once with 1375 g of 70 wt% ethanol aqueous solution and once with 1250 g of 90 wt% ethanol aqueous solution, and alcohol replacement was performed twice with 1250 g of 100 wt% ethanol aqueous solution. Thereafter, the sample was immersed in 1250 g (1 to 100 wt%) of acetic acid-containing ethanol solution or aqueous ethanol solution containing hydrochloric acid (2 to 5 wt%), stirred for 1 hour, and washed once with 1375 g of 70 wt% ethanol aqueous solution and once with 1250 g of 90 wt% ethanol aqueous solution, and alcohol replacement was performed twice with 1250 g of 100 wt% ethanol aqueous solution, and the sample was dried. Further, the sample was placed in a hot-air dryer under the conditions of 105 °C for 6 h or 120 °C for 3 h to obtain a protonated sheet-like structure. The data on the average degree of substitution (DS) and degree of protonation are shown in Table 1 below together with the protonation conditions.

[Table 1]

| Sample | Fiber Material | Average Degree of Substitution | Protonation Reagent | Heat Treatment | Concentration (wt%) | Degree of Protonation (%) |
|---|---|---|---|---|---|---|
| Sample 1 | | | | | 1 | 2.5 |
| Sample 2 | | | | | 2.5 | 5.1 |
| Sample 3 | | | | | 5 | 10.2 |
| Sample 4 | | | | | 10 | 16.6 |
| Sample 5 | | | Acetic Acid | | 40 | 25.8 |
| Sample 6 | | 0.55 | | | 60 | 28.5 |
| Sample 7 | | | | | 80 | 34.5 |
| Sample 8 | | | | | 100 | 39.9 |
| Sample 9 | | | | | 2 | 61.3 |
| Sample 10 | Long Continuous Cupra Fibers (filaments) | | Hydrochloric Acid | | 3 | 79.8 |
| Sample 11 | | | | 105 °C, 6h | 4 | 96.8 |
| Sample 12 | | | | | 5 | 100 |
| Sample 13 | | 0.22 | | | | 50.2 |
| Sample 14 | | 0.31 | | | | 49.9 |
| Sample 15 | | 0.43 | | | 100 | 50.9 |
| Sample 16 | | 0.82 | | | | 48.7 |
| Sample 17 | | 0.96 | | | | 40.1 |
| Sample 18 | | 1.2 | | | | 40.4 |
| Sample 19 | Short Lyocell Fibers (staple fibers) | 0.53 | Acetic Acid | | 40 | 23.4 |
| Sample 20 | Short Rayon Fibers (staple fibers) | 0.54 | | | | 24.2 |

(continued)

| Sample | Fiber Material | Average Degree of Substitution | Protonation Reagent | Heat Treatment | Concentration (wt%) | Degree of Protonation (%) |
|---|---|---|---|---|---|---|
| Sample 21 | Long Continuous Cupra Fibers (filaments) | 0.55 | | 120 °C, 3h | 1 | 2.5 |
| Sample 22 | | | | | 2.5 | 5.1 |
| Sample 23 | | | | | 5 | 10.2 |
| Sample 24 | | | | | 10 | 16.6 |
| Sample 25 | | | | | 40 | 25.8 |

It was discovered that it is possible to control the degree of protonation by controlling the acid type and the acid concentration. It was also discovered that control of the degree of protonation can be performed regardless of the degree of substitution of the raw fabric.

[Reference Example 2]

A total of 10 sections (2 cm × 1 cm) of the CMC sheet-like structure (degree of substitution: 0.55) before protonation obtained by the method of Reference Example 1 were cut and were placed into each 50 mL plastic tube. 30 mL of a hydrochloric acid-containing methanol solution (1.2 mol/L hydrochloric acid, 90% methanol) was added to each tube, and incubated at room temperature for 10 minutes, 30 minutes, or 1 hour.

After incubation, the samples were washed with an 80% methanol aqueous solution and 100% methanol, in this order, and dried to obtain protonated sheet-like structures. Data on the degree of protonation is shown in Table 2 below along with the protonation conditions.

[Table 2]

| Reagent Used | Hydrochloric Acid | | |
|---|---|---|---|
| Concentration (wt%) | 1.2 mol/L (5.5 wt%) | | |
| Immersion Time | 10 min | 30 min | 1 h |
| Degree of Protonation (%) | 98 | 97 | 97 |

It was confirmed that almost 100% of the substituents protonated at the hydrochloric acid concentration of Reference Example 2.

[Example 1]

The liquid absorbencies and strengths of Samples 2 to 10 prepared in Reference Example 1, which differ in degree of protonation, were evaluated. Furthermore, Aquacel (registered trademark, manufactured by CONVATEC Co.), which is a commercially available wound coating agent using CMC, was also evaluated. The evaluation results are shown in Table 3 below.

[Comparative Example 1]

The liquid absorbencies and strengths of Samples 1, 11, and 12 prepared in Reference Example 1, which differ in degree of protonation, were evaluated. Furthermore, Aquacel (registered trademark, manufactured by CONVATEC Co.), which is a commercially available wound coating agent using CMC, was also evaluated. The evaluation results are shown in Table 3 below.

[Table 3]

| Sample | 1 | 2 | 3 | 4 | 5 | 6 | 7 | 8 | 9 | 10 | 11 | 12 | Aquacel |
|---|---|---|---|---|---|---|---|---|---|---|---|---|---|
| Degree of Protonation (%) | 2.5 | 5.1 | 10.2 | 16.6 | 25.8 | 28.5 | 34.5 | 39.9 | 61.3 | 79.8 | 96.8 | 100 | - |
| Strength | Poor | Fair | Good | EXC | EXC | EXC | EXC | EXC | EXC | EXC | EXC | EXC | - |
| Liquid Absorption Amount (g/100 cm$^2$) | 38.1 | 39.2 | 38.1 | 38.9 | 40.0 | 37.3 | 33.2 | 23.1 | 17.8 | 15.4 | 7 | 6.6 | 15.3 |

It was discovered that as the degree of protonation increased, the strength increased and when the degree of protonation was 5% or more, sufficient strength for use was achieved. The liquid absorption amount decreased as the degree of protonation increased. However, it can be understood that when the degree of protonation was 80% or less, the liquid absorption amount was equal to or higher than that of the Aquacel.

[Example 2]

The liquid absorbencies and strengths of Samples 8 and 14 to 17 prepared in Reference Example 1, which differ in degree of substitution, were evaluated. The evaluation results are shown in Table 4 below.

[Comparative Example 2]

The liquid absorbencies and strengths of Samples 13 and 18 prepared in Reference Example 1, which differ in degree of substitution, were evaluated. The evaluation results are shown in Table 4 below.

[Table 4]

| Sample | 13 | 14 | 15 | 8 | 16 | 17 | 18 |
|---|---|---|---|---|---|---|---|
| Average Degree of Substitution | 0.22 | 0.31 | 0.43 | 0.55 | 0.82 | 0.96 | 1.2 |
| Degree of Protonation (%) | 50.2 | 49.9 | 50.9 | 43.6 | 48.7 | 40.1 | 40.4 |
| Strength | EXC | EXC | EXC | EXC | Good | Fair | Poor |
| Liquid Absorption Amount (g/100 cm$^2$) | 6.2 | 15.2 | 17.2 | 27.7 | 30.1 | 30.2 | 31.4 |

It was discovered that as the degree of substitution increased, the strength decreased and when the degree of substitution was 1.0 or more, even with the same degree of protonation, the strength was weak and the sample could not be used. Furthermore, it was discovered that the liquid absorption amount increased as the degree of substitution increased and when the degree of substitution was 0.3 or more, a sufficient liquid absorption amount was obtained.

[Example 3]

The strengths and shape retentions of samples 5, 19, and 20 prepared in Reference Example 1, which differ in fiber material, were evaluated. The results are shown in Table 5 below.

[Table 5]

| Sample | 5 | 19 | 20 |
|---|---|---|---|
| Average Degree of Substitution | 0.55 | 0.53 | 0.54 |
| Degree of Protonation (%) | 25.8 | 23.4 | 24.2 |
| Strength | EXC | EXC | EXC |
| Shape Retention | Good | Fair | Fair |

In all of the nonwoven fabrics, partial protonation brought about sufficient strength. In particular, it was discovered that when the cupra continuous long fiber nonwoven fabric was used, since the entanglement of fibers was strong even when gelled, the shape did not easily collapse even if immersed for a long time.

[Example 4]

The liquid absorbencies and strengths of samples 22 to 25 prepared in Reference Example 1, which had heat treatment conditions of 3 h at 120 °C, were evaluated. The evaluation results are shown in Table 6 below.

[Comparative Example 3]

The liquid absorbency and strength of sample 21 prepared in Reference Example 1, which had heat treatment conditions of 3 h at 120 °C, were evaluated. The evaluation results are shown in Table 6 below.

[Table 6]

| Sample | 21 | 22 | 23 | 24 | 25 |
|---|---|---|---|---|---|
| Degree of Protonation (%) | 2.5 | 5.1 | 10.2 | 16.6 | 25.8 |
| Strength | Fair | Good | EXC | EXC | EXC |
| Liquid Absorption Amount (g/100 cm$^2$) | 41.2 | 40.3 | 41.6 | 37.4 | 30.0 |

It was discovered that by setting the heat treatment conditions to 3 h at 120 °C, when the degree of protonation was 5% or more, sufficient strength was maintained.

INDUSTRIAL APPLICABILITY

By performing wound healing using the sheet for covering a wound containing partially protonated carboxymethyl cellulose of the present invention, wound healing is promoted due to excellent exudate retention. Furthermore, since the sheet can be removed while the form is maintained, few residuals are left in the wound site. Since there is no inflammation of the wound site, it is possible to provide treatment with minimal impact on the human body.

**Claims**

1.   A sheet for covering a wound, which is a wound covering material containing fibers of carboxymethylated natural or regenerated cellulose, wherein the average degree of substitution of hydroxyl groups in a glucose unit constituting the cellulose is in the range of 0.3 to 1.0, and 5 % to 60 % of carboxymethyl groups are protonated.

2.   The sheet for covering a wound according to claim 1, having a thickness in the range of 0.03 mm to 5.0 mm.

3.   The sheet for covering a wound according to claim 1 or 2, having a density of 0.03 g/cm$^3$ to 1.0 g/cm$^3$.

4.   The sheet for covering a wound according to any one of claims 1 to 3, in a cotton-like, woven fabric, or non-woven fabric form.

**Patentansprüche**

1.   Auflage zur Abdeckung einer Wunde, bei der es sich um ein Wundabdeckungsmaterial handelt, das Fasern aus carboxymethylierter natürlicher oder regenerierter Cellulose enthält, wobei der mittlere Substitutionsgrad von Hydroxygruppen in einer Glucoseeinheit, die die Cellulose bildet, im Bereich von 0,3 bis 1,0 liegt und 5% bis 60% der Carboxymethylgruppen protoniert sind.

2.   Auflage zur Abdeckung einer Wunde gemäß Anspruch 1, die eine Dicke im Bereich von 0,03 mm bis 5,0 mm aufweist.

3.   Auflage zur Abdeckung einer Wunde gemäß Anspruch 1 oder 2, die eine Dichte von 0,03 g/cm$^3$ bis 1,0 g/cm$^3$ aufweist.

4.   Auflage zur Abdeckung einer Wunde gemäß einem der Ansprüche 1 bis 3 in einer baumwollartigen Gewebe- oder Vliesstoffform.

**Revendications**

1.   Feuille pour couvrir une plaie, laquelle est un matériau couvrant une plaie contenant des fibres de cellulose naturelle ou régénérée carboxyméthylée, dans laquelle le degré moyen de substitution de groupes hydroxyle dans une unité glucose constituant la cellulose se trouve dans l'intervalle de 0,3 à 1,0, et de 5 % à 60 % de groupes carboxyméthyle sont protonés.

2.   Feuille pour couvrir une plaie selon la revendication 1, présentant une épaisseur dans l'intervalle de 0,03 mm à 5,0 mm.

3. Feuille pour couvrir une plaie selon la revendication 1 ou 2, présentant une densité de 0,03 g/cm$^3$ à 1,0 g/cm$^3$.

4. Feuille pour couvrir une plaie selon l'une quelconque des revendications 1 à 3, dans une forme semblable au coton, de textile tissé, ou de textile non tissé.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013135704 A
- JP 2000256958 A
- JP 2002143210 A
- WO 2011121858 A